# EUROPÄISCHE PATENTSCHRIFT

(11) **EP 0 680 462 B1**
(45) Veröffentlichungstag und Bekanntmachung des Hinweises auf die Patenterteilung: **09.04.1997**
(21) Anmeldenummer: 94903862.4
(22) Anmeldetag: 22.12.1993
(51) Int. Cl.: C07C 13/62, C07C 43/215, C07C 205/45, C07C 69/157, C07C 205/58, C07D 213/55, C07C 225/22, C07C 2/86

(54) **THERMISCH STABILE FULLERENDERIVATE UND VERFAHREN ZU IHRER HERSTELLUNG**
THERMALLY STABLE FULLERENE DERIVATES AND PROCESS FOR PRODUCING THE SAME
DERIVES FULLERENES THERMIQUEMENT STABLES ET LEUR PROCEDE DE PREPARATION

(30) Priorität: 20.01.1993 DE 4301458
(43) Veröffentlichungstag der Anmeldung: 08.11.1995
(73) Patentinhaber: HOECHST AKTIENGESELLSCHAFT, 65926 Frankfurt am Main (DE)
(72) Erfinder: GÜGEL, Andreas, D-55118 Mainz (DE); BELIK, Pavel, D-63456 Hanau (DE); MÜLLEN, Klaus, D-50939 Köln (DE)
(86) Internationale Anmeldenummer: EP9303658
(87) Internationale Veröffentlichungsnummer: WO9417018

(56) Entgegenhaltungen:
- EP-A- 0 546 718
- G.S. HAMMOND et al., "Fullerenes", ACS Symposium Series 481, Kapitel 11: "Survey of Chemical Reactivity of C60' Electrophile and Dieno-polar- ophile Par Excellence" AMERICAN CHEMICAL SOCIETY, Washington D.C., 1992, Seiten 161-175 insbesondere Seite 163 (in der Beschreibung genannt).
- CHEMICAL REVIEWS, Band 70, Nr. 4, ver!ffentlicht 1970 I.L. KLUNDT "Benzocyclobutene and its derivatives" Seiten 471-487
- CHEMICAL REVIEWS, Band 70, Nr. 4, ver!ffentlicht 1970 I.L. KLUNDT "Benzocyclobutene and its derivatives" Seiten 471-487 insbesondere Seiten 471-473(in der Beschreibung genannt)

## Beschreibung

Fullerene sind käfigförmige Kohlenstoffallotrope der allgemeinen Formel (c₂₀₊₂ₘ) (mit m = natürliche Zahl). Sie enthalten zwölf Fünf- sowie beliebig viele, mindestens aber zwei Sechsringe aus Kohlenstoffatomen.

Obwohl diese Verbindungsklasse erst 1985 von Kroto und Smalley nachgewiesen (Nature, 1985, 318, 162) wurde und Krätschmer und Huffmann erst 1990 über die Darstellung makroskopischer Mengen an C₆₀ berichteten (Nature, 1990, 347, 354), sind solche Verbindungen sehr schnell auf ein breites Interesse gestoßen und wurden innerhalb kürzester Zeit Gegenstand zahlreicher Forschungsarbeiten ( siehe z.B. G.S. Hammond, V.J. Kuck (Editors), "Fullerenes", ACS Symposium Series 481, American Chemical Society, Washington DC 1992 und Accounts of Chemical Research 1992, 25, 98-175).

Der Einsatz von Fullerenen als Synthesebausteine in der organischen Chemie beinhaltet große Schwierigkeiten, da sich z.B. der Substitutionsgrad von Fullerenen bei der Derivatisierung nur schwer steuern läßt. C₆₀ enthält 30 Doppelbindungen, die prinzipiell als Reaktionszentren zur Verfügung stehen. Zudem ist eine sehr große Zahl von Stellungsisomeren möglich, sobald auch nur zwei Substituenten am Fullerengrundkörper vorhanden sind.

Weitere Probleme bereitet die geringe Löslichkeit der Fullerene in allen gängigen Lösungsmitteln.

Da man aber ein hohes Potential dieser Stoffklasse, beispielsweise im Bereich der Optoelektronik und Wirkstofforschung erwartet, wurden bereits Anstrengungen zur Derivatisierung, insbesondere von C₆₀, unternommen (siehe z.B. H. Schwarz, Angew.Chemie. 1992, 104, 301 und F. Wudl et al. in ("Fullerenes" G.S. Hammond, V.S. Kuck, eds. ACS Symp. Ser. 481, S. 161, Washington DC 1992 und Accounts of Chemical Research, 1992, 25, 157.

Es ist bereits bekannt, daß Fullerene mit Dienen zu Diels-Alder-Addukten umgesetzt werden können ("Fullerenes" ACS-Symposium Series No. 481, S. 164). Die dienophile Reaktivität des C₆₀ wurde bisher nur in der Reaktion mit Anthracen und Furan sowie Cyclopentadien beobachtet. Alle diese Reaktionen zeichnen sich jedoch durch eine breite Produktverteilung aus. Weiterhin zerfallen diese Produkte beim Erwärmen wieder in ihre Ausgangsverbindungen.

Es ist weiterhin bekannt, Fullerene in einer 1,3 dipolaren Cycloaddition mit Diazoverbindungen umzusetzen (z.B. F. Wudl, Acc. Chem. Res., 1992, 25, 157).

Wünschenswert war es, Derivate der Fullerene zu synthetisieren, welche thermisch stabil sind, das Fullerengrundgerüst enthalten, und weitere Reaktionen an der derivatisierten Stelle ermöglichen.

Es wurde nun gefunden, daß sich wohldefinierte Fullerenderivate erhalten lassen, indem Fulleren mit einer Verbindung der Formel 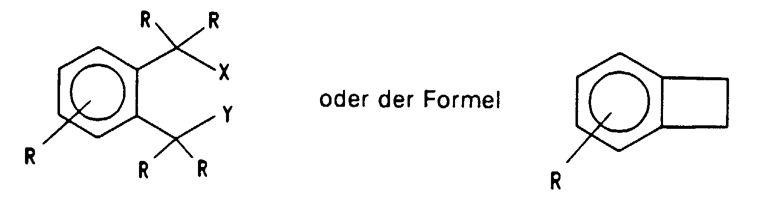 umgesetzt werden, wobei die Anbindung des aromatischen Ringsystems über einen cycloaliphatischen 6-Ring an das Fulleren erfolgt.

Gegenstand der Erfindung ist somit ein Fullerenderivat der Formel I, 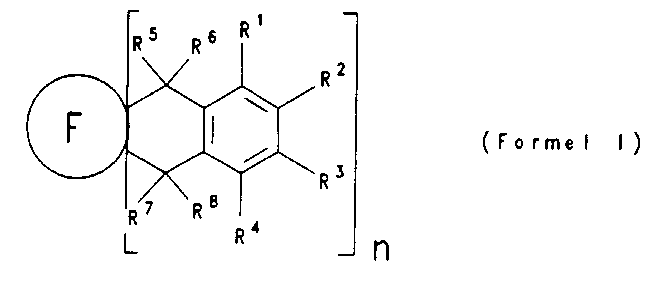 wobei die Symbole und Indices folgende Bedeutung haben:
- Ⓕ: ist ein Fullerenrest der Formel (C₂₀₊₂ₘ), wobei m die Zahl 1 bis 50 darstellt
- R¹ bis R⁸: ist gleich oder verschieden H, NH₂, NR⁹R¹⁰, NR⁹H, CO₂R⁹, OCOR¹⁰, CN, COR¹⁰, Cl, Br, J, F, OR¹¹, CONH₂, C₂-C₂₀ Alkyl, das mit Cl, J, Br und F substituiert sein kann, C₃-C₈ Cycloalkyl, Aryl, Heteroaryl, wobei R⁹ bis R¹¹ H, C₁-C₂₀ Alkyl, welches durch F, Cl, Br oder J substituiert sein kann, Pyridinyl oder Phenyl ist, welches seinerseits durch F, Cl, Br, J, Nitro, Amino, C₁-C₂₀-Alkylamin, C₆-C₁₄-AryDamin, C₁-C₂₀-Alkoxy oder C₆-C₁₄-Aryloxy substituiert sein kann, oder -(CH₂)ⱼ-CO₂H mit j = 1 bis 10 ist, R¹-R⁴ und/oder R⁵,R⁷ können auch Teil eines cyclo-aliphatischen, aromatischen oder heteroaromatischen Systems sein, welches seinerseits mit C₁-C₂₀ Alkyl, Aryl, Carboxyl, Carbonyl, Alkoxy, Aryloxy, F, Cl, Br, J, Nitro, Alkohol oder Amin substituiert ist, oder R¹ und R², R² und R³, R³ und R⁴ können jeweils gemeinsam 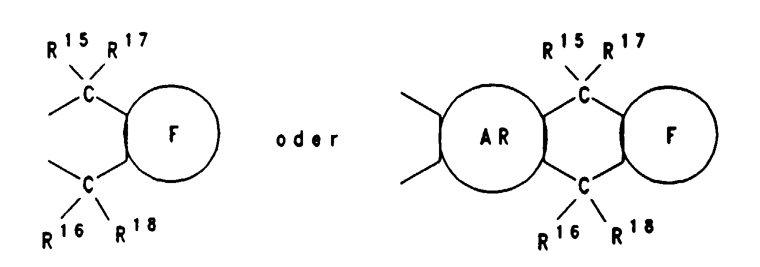 sein, wobei R¹⁵ - R¹⁸ H, C₁-C₂₀ Alkyl, F, Cl, Br, J oder Phenyl, und 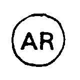 den Rest eines annelierten aromatischen Systems darstellen,
- R⁵ bis R⁸: kann auch das Strukturelement der Formel V 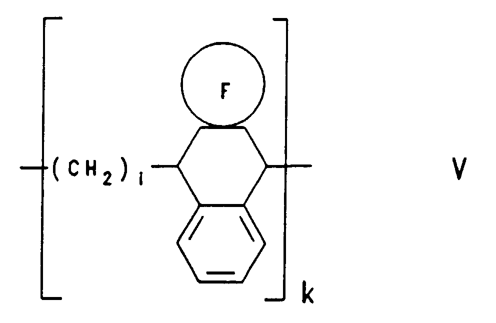 sein, in der i eine Zahl von 2 bis 20 und k eine Zahl größer 1 ist, und R⁵ und R⁷ zusammen können auch eine Brücke aus -O- sein,
- n: ist 1 bis 20.

Bevorzugt sind Verbindungen der Formel I, in denen die Symbole und Indices folgende Bedeutung haben:
- Ⓕ: ist ein Fullerenrest der Formel (C₂₀₊₂ₘ), in dem m 20, 25, 28, 29, 31 oder 32 ist,
- R¹ bis R⁸: haben die oben genannte Bedeutung,
- n: ist 1 oder 2.

Besonders bevorzugt sind Verbindungen der Formel I, in denen die Symbole und Indices folgende Bedeutung haben:
- Ⓕ: ist C₆₀ oder C₆₀
- R¹ bis R⁴: sind gleich oder verschieden und bedeuten
H, NH₂, COR⁹, CO₂R¹⁰, -O-R¹¹, OCOR¹⁰ oder C₁-C₁₀ Alkyl, das mit F, Cl, Br oder J substituiert sein kann, wobei R⁹ bis R¹¹ H, (C₁-C₁₀)-Alkyl, das mit F, Cl oder Br substituiert sein kann, Pyridinyl, Phenyl, welches durch F, Cl, Br, Nitro, Amino, C₁-C₁₀-ACkySamin, C₆-C₁₄-AryDamin, C₁-C₁₀-Alkoxy oder C₆-C₁₄-Aryloxy substituiert sein kann, oder -(CH₂)ⱼ -CO₂H mit j= 1 bis 10, ist, oder R² und R³ zusammen 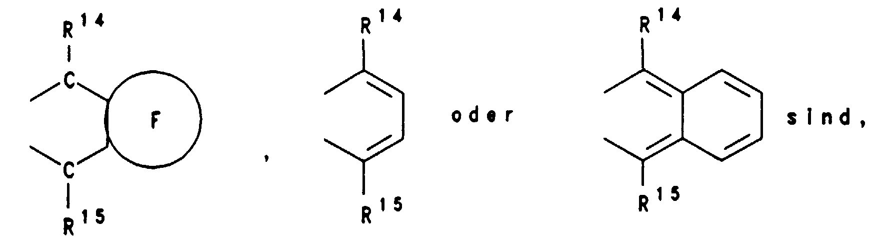 wobei R¹⁴ und/oder R¹⁵ H, Phenyl oder (C₁-C₁₀)-Alkyl ist, und
- R⁵ bis R⁸: gleich oder verschieden H, F, Cl, Br, (C₁-C₁₂)-Alkyl oder ein Strukturelement der Formel V mit i = 4 bis 12 und k eine Zahl größer 1 ist und
- n: 1 oder 2 ist.

Insbesondere bevorzugt sind Verbindungen der Formel I, in denen die Symbole und Indices folgende Bedeutung haben:
- Ⓕ: ist C₆₀ und
- R¹ bis R⁴: sowie R⁵ bis R⁸ haben die zuletzt genannte Bedeutung.

Die Bindung des Strukturelements 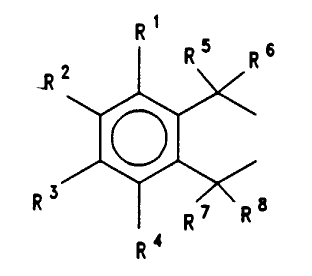 aus Formel I an das Fulleren erfolgt über zwei benachbarte C-Atome im FullerenMolekül, die wiederum als Strukturelement durch die Formel II a und II b wiedergegeben werden können

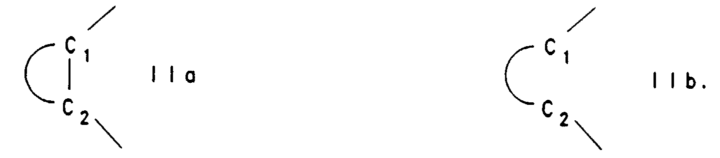

Die erfindungsgemäßen Verbindungen der Formel I werden beispielsweise durch Cycloaddition der Verbindungen III oder IV an ein Fullerenmolekül hergestellt.

Dies kann prinzipiell nach literaturbekannten Methoden zu Addition eines Dien an Olefine erfolgen, wie beispielsweise bei I.L.Klundt, Chemical Reviews, 1970, Vol. 70, No.4, 471-487 und R.P. Thummel, Acc. Chem. Res. 1980, 13, 70-76 beschrieben. Bis-Brommethyl-Benzol-Derivate (III) können wie z.B. bei I.L. Klundt, Chemical Reviews, 1970, Vol. 70, No. 4,471-487 und R.P. Thummel, Acc. Chem. Res. 1980, 13, 70-76 beschrieben hergestellt werden. 1,3-Dihydroisothianaphten-2,2-dioxid-Derivate (III, Y = SO₂), Benzocyclobutenderivate IV werden gemäß A.P. Covc J.Org. Chem. 1969, 34, 538 bzw. J. A. Chem. Soc. 1959, 81, 4266 hergestellt.

Bevorzugt ist ein Verfahren zur Darstellung von Verbindungen der Formel I, bei dem ein Fulleren der allgemeinen Formel C₍₂₀₊₂ₘ₎ (m = 0, 1, 2, ...) in einem aprotischen organischen Lösungsmittel, wie Toluol, CCL₄, CH₂Cl₂, Benzol, Chlorbenzol mit substituierten Ortho-Alkyl-Benzolen der Formel III, 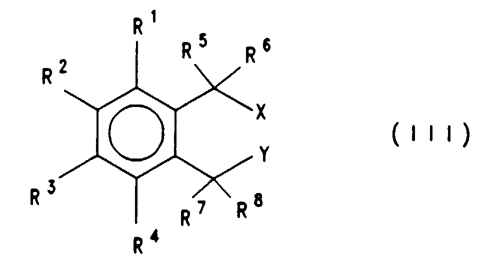 in der R¹ bis R⁸ die obengenannte Bedeutung haben und X und Y jeweils Halogen bedeuten oder zusammen SO₂, CO₂ oder N₂ darstellen, oder aber Verbindungen der Formel IV, 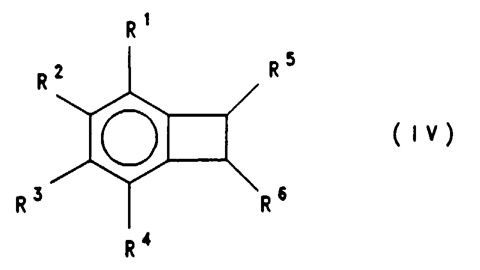 in der R¹ bis R⁶ die obengenannte Bedeutung haben, umgesetzt wird.

Die Reaktion kann in einem Temperaturbereich von 100 bis 300°C, vorzugsweise 100 bis 210°C, durchgeführt werden. Zur Darstellung von Verbindungen der Formel I mit n = werden die Ausgangsverbindungen bevorzugt in stöchiometrischen Mengen eingesetzt.

Als Fulleren werden insbesondere bevorzugt reines C₆₀ und/oder C₆₀ eingesetzt aber auch Rohfullerene, die ein Gemisch aus C₆₀ und C₆₀ als Hauptkomponenten enthalten. Es können aber auch alle anderen denkbaren Fullerene bzw. Fullerenderivate eingesetzt werden.

Die Fullerene sind zum Teil im Handel erhältlich oder können durch Herstellung von Fullerenruß im Lichtbogenverfahren mit anschließender Extraktion mit einem unpolaren organischen Lösungsmittel (Rohfullerene), wie z.B. in WO 92/09279 beschrieben, gewonnen werden. Die weitere Feinauftrennung kann säulenchromatographisch erfolgen.

Die erfindungsgemäßen Verbindungen der Formel I können in optoelektronischen Bauelementen Anwendung finden.

Die Erfindung wird durch die folgenden Beispiele näher erläutert.

### Beispiele:

Umsetzung von Fulleren mit ortho-Chinodimethan-Derivaten:
A. Umsetzung von C₆₀ via baseninduzierter 1,4-Eliminierung von 1,2-Bis(brommethyl)-Aromaten; allgemeine Arbeitsvorschrift:
   C₆₀ (100 mg = 0.139 mmol), 1,2-Bis(brommethyl)-Aromat (0.139 mmol), Kl (50 mg = 0.3 mmol) und [18]Krone-6 (300 mg = 1.13 mmol) in 50 mL Toluol werden mehrere Stunden erhitzt, wobei sich die Lösung verfärbt. Das nach gängiger Aufarbeitung erhaltene Rohprodukt läßt sich z.B. chromatographisch reinigen.
   A.1: m = 10; n = 1; R¹ - R⁸ = H 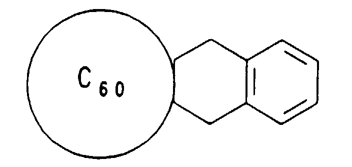 Reaktionsdauer: 6 h
      Ausbeute 88 % (bezogen auf umgesetztes C₆₀)
      Charakterisierung:
      ¹H-NMR (500 MHz, C₂Cl₄D₂, 28°C): δ = 4.46 ( d verbreitert, 2 H; CH₂); 4.82 (d verbreitert, 2 H; CH₂); 7.57 - 7.59 u. 7.69 - 7.71 (m, AA'BB'-System des Arens); Messung bei 125°C: δ = 4.70 (s, 4 H; CH₂);
      ¹³C-NMR (125 MHz, C₂Cl₄D₂, 80°C, J-moduliertes Spin-echo für ¹³C): δ = 45.28 (Benzyl-C); 66.09 (aliphat. quarternäre C₆₀ C-Atome); 128.02 u. 128.09 (H-subst. aromat. C-Atome), 135.76, 138.21, 140.20, 141.66, 142.13, 142.30, 142.62, 143.17, 144.78, 145.42, 145.49, 145.51, 145.85, 146.30, 146.52, 147.76 u. 156.93 (17 Aren-Signale).
      MS(FD): m/z 824
      UV/VIS (CHCl₃): C₆₀-ähnlich, jedoch weniger strukturiert; schwaches Maximum bei 435 nm.
   A.2: m= 20; n = 1; R¹, R⁴ = CH₃; R², R³, R⁵-R⁸ = H 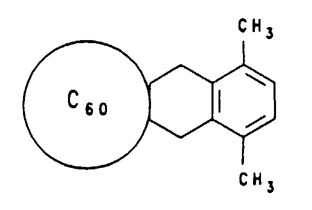 Reaktionsdauer: 8 h
      Ausbeute: 86% (bezogen auf umgesetztes C₆₀)
      Charakterisierung:
      ¹H-NMR (500 MHz, C₂Cl₄D₂, 28°C), δ = 2,58 (s, 6H, CH₃); 4.09(verbreitert, 4H; CH₂); 7,32 (s, 2H des Arens);
      ¹³C-NMR (125 MHz, C₂D₂Cl₄, 28°C), δ = 29,8 (Methyl), 41,2 (CH₂), 66 (aliphat. quarternäre C₆₀-C-Atome), 128,9 (H-subst. aromat. C-Atome); 133,2; 136,7; 140,2; 141,6; 142,1; 142,3; 142,6; 143,1; 144,8; 145,5; 146,3; 146,5; 147,7; 156,8
      MS (FD): m/z 852
      UV/VIS (CHCl₃): C₆₀ -ähnlich, jedoch weniger strukturiert; schwaches Maximum bei 435 nm.
   A.3: m = 20; n = 1, R¹, R³ - R⁸, = H; R² = OCH₃; 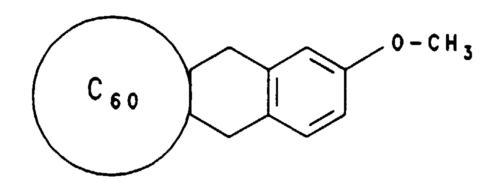 Reaktionsdauer: 12 h
      Ausbeute: 82% (bezogen auf umgesetztes C₆₀)
      Charakterisierung:
      ¹H-NMR (500 MHz, C₂Cl₄D₂, 28°C), δ = 3,97 (s; OCH₃); 4,41-4,55 (m; verbreitert; CH₂); 4,76-4,80 (m; verbreitert; CH₂);
      7,08-7,10 (dd; aren-H); 7,26 (d; aren-H); 7,60-7,62 (d, aren-H).
      MS (FD): m/z 854
      UV/VIS (CHCl₃): C₆₀ -ähnlich, jedoch weniger strukturiert; schwaches Maximum bei 435 nm.
B: via thermischer SO₂-Extrusion aus Verbindungen der Formel III
   Beispiel B.1: m = 20; n = 1; R¹ - R⁸ = H;
      Versuchsvorschrift:
      C₆₀ (75 mg = 0.104 mmol), und 1,3-Dihydro-isothianaphthen-2,2-dioxid (25 mg = 0.149 mmol) in 20 ml 1,2,4-Trichlorbenzol werden 2 Tage zum Rückfluß erhitzt, wobei sich die Reaktionslösung braun färbt. Man versetzt mit 40 ml Ethanol und filtriert das ausgefallene braune Reaktionsprodukt ab. Das Reaktionsprodukt kann z.B. mittels Chromatographie über mit Divenylbenzolquervernetzten Polystyrolgel (100 Å, 5µm) mit CHCl₃ oder Toluol als Eluenten in nicht umgesetztes C₆₀, mono-Additionsprodukt (n = 1) und sehr kleine Mengen bis-Additionsprodukt (n = 2) getrennt werden. Die Ausbeute an mono-Additionsprodukt (n = 1) beträgt 81% (bezogen auf umgesetztes C₆₀). Zur Charakterisierung siehe Beispiel A.1.
   Beispiel B.2: m = 20; n = 1; R¹, R⁴ = Phenyl; R⁵ - R⁸ = H; R², R³ = Teil eines ankondensierten Benzolrings
      Versuchsvorschrift:
      C₆₀ (80 mg = 0.111 mmol) und 4,9-Diphenyl-1,3-dihydronaphtho [2,3-c] thiophen-2,2-dioxid 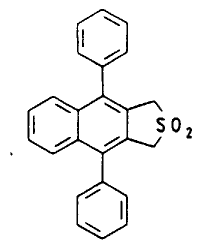 (62 mg = 0.167 mmol) in 20 ml 1,2,4-Trichlorbenzol werden für 3 Tage zum Rückfluß erhitzt, wobei sich die Reaktionslösung braun färbt. Die Reaktion wird analog zu Beispiel B. 1 aufgearbeitet.
   Die Ausbeute an mono-Additionsprodukt (n = 1) beträgt 75% (bezogen auf umgesetztes C₆₀)
   Charakterisierung des mono-Additionsprodukts (n = 1):
   FD-Massenspektroskopie: 1025.98 (100%, M⁺)
   ¹H-NMR (500 MHz, CDCl₃), 4.47 ppm (d, 2H); 4.75 ppm (d, 2H); 7.22-7.7 ppm (14H)
   ¹³C-NMR (125 MHz, CDCl₃): 42.4 ppm (CH₂); 65.72 ppm (quarternäres aliphatisches C-Atom von C₆₀)
   UV (CHCl₃): schwaches Absorptionsmaximum bei 432.5 nm (charakteristisch für C₆₀-mono-Additionsprodukte)
C: via thermischer Ringöffnung von Benzocyclobutenderivaten der Formel IV
   Beispiel C.1: m = 20; n = 1; R¹ - R⁸ = H;
      Versuchsvorschrift:
      C₆₀ (100 mg = 0.139 mmol) und Benzocyclobutan 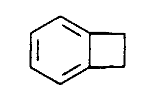 (Herstellung nach P.Schiess et al. Tetrahedron Letters 1982, 23,3665)(16 mg = 0.154 mmol) in 20 ml 1,2-Dichlorbenzol werden 3 Stunden zum Rückfluß erhitzt, wobei sich die Reaktionslösung braun färbt. Das Reaktionsgemisch wird analog zu Beispiel B.1 aufgearbeitet. Die Ausbeute an Mono-Additionsprodukt (n = 1) beträgt 90% (bezogen auf umgesetztes C₆₀). Zur Charakterisierung siehe Beispiel A.1.
   Beispiel C.2: m = 20; n = 1; R⁵, R⁷ = Br; R¹ - R⁴, R⁶, R⁸, = H;
      Versuchsvorschrift:
      C₆₀ (100 mg = 0.139 mmol) und 1,2-Dibrombenzocyclobutan 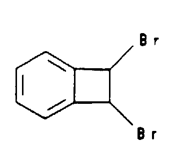 (Herstellung nach M.P.Cava et al. J. Am.Chem.Soc. 1959, 81,6458)(40.5 mg = 0.155 mmol) in 20 ml 1,2-Dichlorbenzol werden 2 Stunden auf 130°C erhitzt, wobei sich die Reaktionslösung braun färbt. Das Reaktionsgemisch wird analog zu Beispiel B.1 aufgearbeitet.
      Die Ausbeute an Mono-Additionsprodukt (n = 1) beträgt 72% (bezogen auf umgesetztes C₆₀).
      Charakterisierung des Mono-Additionsprodukts (n = 1):
      FD-Massenspektroskopie: 981.7 (100%, M⁺); 901.8 (45%, M⁺.Br); 821.9 (87%, M⁺.Br₂)
      ¹H-NMR (500 MHz, CDCl₃), 6.96 ppm (s, 1H, CHBr); 7.66 ppm (t, 1H, CH); 7.76 ppm (s+t, 2H, CH); 8.04 ppm (s, 1H, CHBr); 8.24 ppm (d, 1H, CH)
      ¹³C-NMR (125 MHz, CDCl₃): 56.26 ppm (CHBr); 57.07 ppm (CHBr); 127.13 ppm (CH-aromatisch); 129.22 ppm (CH-aromatisch); 130.45 ppm (CH-aromatisch); 131.01 ppm (CH-aromatisch); 155.14, 152.61, 152.53, 151.74, 147.87, 147.77, 147.75, 147.54, 146.75, 146.60, 146.57, 146.56, 146.36, 146.26, 146.15, 146.08, 145.84, 145.83, 145.59, 145.54, 145.50, 145.43, 145.35, 145.30, 144.78, 144.63, 144.37, 144.02, 144.00, 143.97, 143.12, 142.84, 142.78, 142.70, 142.68, 142.59, 142.52, 142.37, 142.31, 142.12, 141.69, 141.51, 141.50, 141.42, 141.41, 141.38, 141.32, 139.97, 138.53, 138.12, 136.64, 136.17, 136.16, 136.02, 135.37 ppm (55 quartäre nicht aliphatische Kohlenstoffatome).
      UV (CHCl₃): schwaches Absorptionsmaximum bei 432.5 nm
   Beispiel C.3: m = 20; n = 1; R¹, R³, R⁴, R⁵, R⁶ = H; R² = 4-Fluor-3-nitrobenzoyl 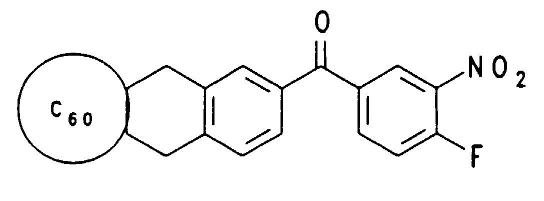 1055 mg C₆₀ (1.465 mmol) und 336 mg 4-(4-Fluor-3-nitrobenzoyl)benzocyclobuten (1.2398 mmol) in 200 ml 1,2,4-Trichlorbenzol wurden 7 h zum Rückfluß erhitzt. Das Lösungsmittel wurde im Vakuum (20 mbar, 130°C) abdestilliert, der Destillationsrückstand in 200 ml CHCl₃ suspendiert, von nicht gelöstem C₆₀ abfiltriert und chromatographisch aufgearbeitet. Man erhielt 72 % des Mono-Addukts, 19 % des Bis-Addukts und 9 % des unumgesetzten C₆₀.
      (n = 1):
      MS (FD: m/z 991.1 ([M] +, 100 %)
      ¹H-NMR (500 MHz, C₂D₂Cl₄, 28°C): d = 4.48 (d (b), 2H, CH₂), 4.81 (d (b), 2H, CH₂), 7.45 (t, 1H, 9 und 10 Hz, i), 7.8 (d, 1H, 8Hz, f), 7.89 (d, 1H, 8 Hz, g), 8.04 (s (b), 1H, e), 8.18 (m, 1H, h), 8.53 ppm (dd, 1H, 2 und 7 Hz, k) ¹³C-NMR (125 MHz, C₂D₂Cl₄, 28°C; J-moduliertes Spinecho für ¹³C): d = 47.97, 48.19 (CH₂), 68.51, 68.56 (sp³-C₆₀-Kohlenstoffatome), 122.08, 122.25, 128.49, 131.23, 131,40, 131.66, 132.23, 132.29, 132.83 (b), 133.06, 140.10, 140.18 (alle CH), 119.4, 119.65, 126.65, 136.75, 137.5, 137.52, 138.69, 141.06, 142.20, 143.20 (b, mehrere Signale überlagert), 144.69 (b, mehrere Signale überlagert), 145.11 (b, mehrere Signale überlagert), 145.17, 145.19, 145.64, 146.16 (b, mehrere Signale überlagert), 147.42, 147.72, 147.75, 148.52 (b, mehrere Signale überlagert), 149.32, 149.53, 149.55, 150.75, 150.77 (alles quartäre nicht aliphatische Kohlenstoffatome), 195.90 ppm (CO)
      UV/VIS (CHCl₃): schwaches Absorptionsmaximum bei ca. 435 nm.
   Beispiel C.4: m = 20; n = 1; R⁵ = OCOCH₃, R¹ - R⁴ = H, R⁶ - R⁸ = H 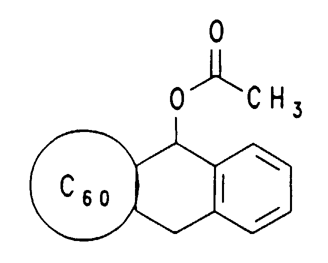 400 mg (2,37·10⁻³ mol) ±-Essigsäurebenzocyclobuten-7-ol-ester wurden mit 813 mg (1,13·10⁻³ mol) C₆₀-Fulleren in 75 ml 1,2,4-Trichlorbenzol 5 Stunden unter Rückfluß erhitzt. Anschließend wurde das Lösungsmittel abdestilliert und der erhaltene Feststoff in Chloroform gelöst. Die Lösung wurde durch ein 0,25 µm Teflonmembranfilter filtriert und chromatographisch über Polystyrolgel gereinigt.
      Ausbeute: 170 mg Mono-, 648 mg Bis- und 374 mg Trisaddukt.
      UV/VIS: (n = 1) zeigt eine Absorptionsbande bei 430 nm.
      FD-Massenspektroskopie: M/Z = 882.2 (M⁺ von (n=1), 66 %), 1044.3 (M⁺ von (n=2), 100 %), 1206.3 (M⁺ von (n =3), 68 %), 1369.5 (M⁺ von (n=4), 4 %)
      (n = 1) (Gemisch zweier Diastereomerer (a) und (b)): ¹H-NMR (500 MHz, C₂D₂Cl₄, 28°C): δ = 2.283 (s, 3H, CH₃, a), 2.288 (s, 3H, CH₃, a'), 4.33 (d, 1H, CH₂, 14 Hz, b), 4.45 (d, 1H, CH₂, 14 Hz, b'), 4.83 (d, 1H, CH₂, 14 Hz, c), 5.31 (d, 1H, CH₂, 14 Hz, c'), 7.21 (s, 1H, CHOR, d), 7.36 (s, 1H, CHOR, d'), 7.49-7.64 (m, 2x4H), CH, e)
      ¹³C-NMR (500 MHz, C₂D₂Cl₄, 28°C; J-moduliertes Spinecho für ¹³C): δ = 44.20, 44.56 (aliphatische, sekundäre C-Atome), 64.31, 66.37, 69.51, 69,79 (aliphatische, quarternäre C₆₀-Atome, Diastereomere), 120.59, 121.58 (quarternäre aromatische C-Atome), 128.09, 128.53, 130.00, 131.22, 131.27, 132.37 (ternäre aromatische C-Atome), 134.22, 137.59, 137.65 (quarternäre aromatische C-Atome), 141.78, 141.87, 142.10, 142.27, 142.72, 142.78, 145.53, 145.59, 145.62, 145.77, 145.84, 146.38, 146.57, 146.64, 146.71, 152.55, 155.75, 156.97, 161.64 (quarternäre C₆₀-Atome, jeweils mehrere Signale überlagert), 170.57, 170.75 (C = O)
   Beispiel C.5: m = 20; n = 1; 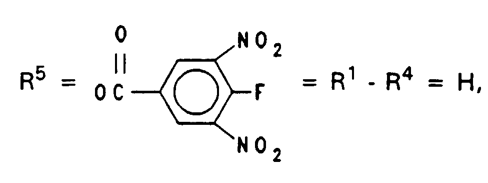 R⁶ - R⁸ = H 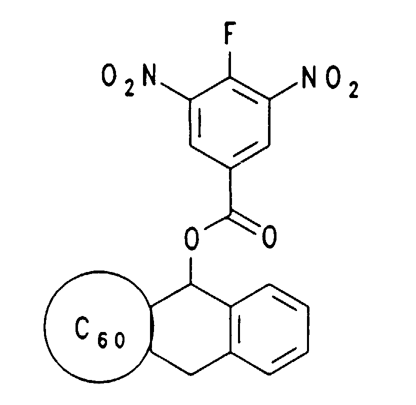 400 mg (1,2·10⁻³ mol) ±-4-Fluor-3,5-dinitrobenzoesäurebenzocylobuten-7-olester wurden zusammen mit 596 mg (8,28·10⁻⁴ mol) C₆₀-Fulleren in 75 ml 1,2,4-Trichlorbenzol 5 Stunden unter Rückfluß erhitzt. Anschließend wurde das Lösungsmittel abdestilliert und der erhaltene Feststoff in Chloroform gelöst. Die Lösung wurde durch ein 0,25 µm Teflonmembranfilter filtriert. Die Trennung der Addukte erfolgte chromatographisch über Polystyrolgel.
      Auf diese Weise konnte 270 mg Mono-, 184 mg Bis- und 95 mg Trisaddukt isoliert werden.
      UV/VIS: (n = 1) zeigt eine Absorptionsbande bei 430 nm.
      FD-Massenspektroskopie: M/Z = 1052 (M⁺ von (n=1), 78 %), 1384 (M⁺ von (n=2), 100 %), 1716 (M⁺ von (n=3), 16 %)
      (n=1) (Gemisch zweier Diastereomerer (a) und (b)): ¹³C-NMR (500 MHz, CDCl₃, J-moduliertes Spinecho): 6 = 44.02, 44.82 (aliphatische, sekundäre C-Atome), 63.84, 66.14, 68.17, 69.22 (aliphatische, quarternäre C₆₀-Atome, Diastereomere), 120.59, 121.58 (quarternäre aromatische C-Atome), 128.09, 128.53, 130.00, 131.22, 131.27, 132.37 (ternäre aromatische C-Atome), 134.22, 137.59, 137.65 (quarternäre aromatische C-Atome), 141.78, 141.87, 142.10, 142.27, 142.72, 142.78, 145.53, 145.59, 145.62, 145.77, 145.84, 146.38, 146.57, 146.64, 146.71, 152.55, 155.75, 156.97 (quarternäre C₆₀-Atome, jeweils mehrere Signale überlagert), 161.64 (C=O)
      (n=1): ¹H-NMR (500 MHz, CDCl₃, 28°C): δ = 4.58 (d, 1H, CH₂, 14 Hz, a), 4.64 (d, 1H, CH₂, 16 Hz, a'), 4.99 (d, 1H, CH₂, 14 Hz, b'), 5.4 (d, 1H, CH₂, 14 Hz, b), 7.55-7.85 (m, 4H, CH, c), 8.01 (s, 1H, CHOR, d), 8.03 (s, 1H, CHOR, d'), 9.12 (s, 2H, CH, e), 9.25 (s, 2H, CH, e')
   Beispiel C.6: m = 20; n = 1; 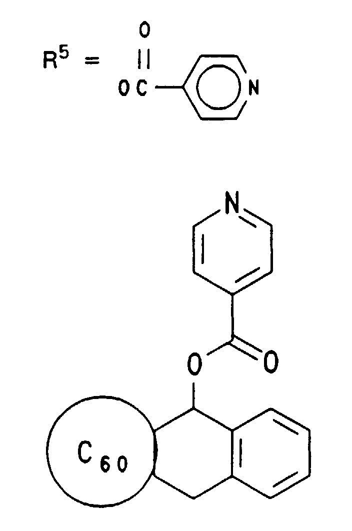 80 mg (3,55·10⁻⁴ mol) ±-Isonicotinsäurebenzocyclobuten-7-olester wurden zusammen mit 500 mg (6,94·10⁻⁴ mol) C₆₀-Fulleren in 75 ml 1,2,4-Trichlorbenzol 5 Stunden unter Rückfluß erhitzt. Anschließend wurde das Lösungsmittel abdestilliert und der erhaltene Feststoff in Chloroform gelöst. Die Lösung wurde durch ein 0,25 µm Teflonmembranfilter filtriert. Anschließend wurden die Addukte über Polystyrolgel getrennt. Ausbeute: 288 mg Mono- und 648 mg Bisaddukt.
      UV/VIS: (n = 1) zeigt eine Absorptionsbande bei 430 nm.
      FD-Massenspektroskopie: M/Z = 945.1 (M⁺ von (n=1), 100 %), 1170.2 (M⁺ von (n=2), 20 %), 1395.3 (M⁺ von (n=3), 2 %)
      (n=1): ¹³C-NMR (500 MHz, C₂D₂Cl₄, 28°C, J-moduliertes Spinecho): δ = 44.5 (aliphatische, sekundäre C-Atome), 64.23 (aliphatische, quarternäre C₆₀-Atome), 123.43, 129.35, 128.34, 130.40, 131.12 (CH, aromatische C-Atome), 134.78, 137.98, 140.23, 140.40, 140.61, 140.65, 141.76, 141.82, 141.92, 142.08, 142.28 (mehrere Signale überlagert), 142.82 (mehrere Signale überlagert), 142.88, 142.93 (mehrere Signale überlagert), 143.34, 143.38, 144.96 (mehrere Signale überlagert), 145.01 (mehrere Signale überlagert),
      145.40 (mehrere Signale überlagert), 145.64, 145.72, 145.76, 145.82, 145.89, 145.91, 145.96, 146.81 (mehrere Signale überlagert), 148.05 (mehrere Signale überlagert), 151.25, 153.18, 153.76, 157.19, 157.27 (alles quarternäre C₆₀-Atome), 151.10 (aromatische, dem Stickstoff im Pyridinrest benachbarte C-Atome), 163.5 (C=O)
      (n=1): ¹H-NMR (500 MHz, C₂D₂Cl₄, 28°C, Gemisch aus zwei Diastereomeren (a) und (b)): 6 = 4.52 (d, 1H, CH₂, 14 Hz, a), 4.58 (d, 1H, CH₂, 14 Hz, a'), 4.97 (d, 1H, CH₂, 14 Hz, b'), 5.44 (d, 1H, CH₂, 14 Hz, b), 7.60-7.67 (m, 4H, CH, c), 7.72 (s, 1H, CHOR, d), 7.74 (s, 1H, CHOR, d'), 8.00 (d, 2H, CH, 6 Hz, e'), 8.12 (d, 2H, CH, 6 Hz, e), 8.81 (d, 2H, CH, 5 Hz, f), 8.89 (d, 2H, CH, 6 Hz, f)
Beispiel D: via thermischer CO₂-Extrusion aus Verbindungen der Formel III
   Beispiel D.1: m = 20; n = 1; R¹, R⁴ - R⁸ = H; R², R³ = OCH₃; X = O; Y = C=O 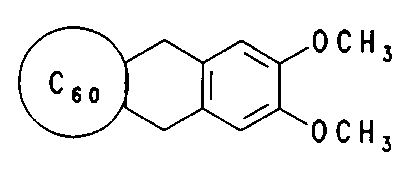 127 mg 6,7-Dimethoxy-3-isochromanon (0.65 mmol) und 360 mg C₆₀-Fulleren (0.5 mmol) in 80 ml 1,2,4-Trichlorbenzol wurden 24 Stunden zum Rückfluß erhitzt. Das Lösungsmittel wurde abdestilliert und das Gemisch chromatographisch aufgearbeitet. Man erhielt 47 % des Mono-Addukts, 22 % des Bis-Addukts und 30 % des nicht umgesetzten C₆₀.
   (n=1):
   MS (FD): m/z 887.4 ( [M]⁺ von (n=1), 100), 903 ([M+16]⁺, 5)
   ¹H-NMR (500 MHz, C₂D₂Cl₄, 28°C): d = 4.01 (s, 6H, -OCH₃), 4.37 (d(b), 2H, CH₂, 4.74 (d(b), 2H, CH₂), 7.20 (s, 2H)
   ¹³C-NMR (125 MHz, C₂D₂Cl₄, 28°C; J-moduliertes Spinecho für ¹³C):
   d = 58,2 (CH₃), 46.62 (CH₂), 67.94 (sp3-C₆₀-Kohlenstoffatome), 113.57 CH, aroamtisch), 132.06, 141.83 (b); 143.33, 143.80, 144.02, 144.29, 144.84, 146.45 (b), 147.18, 147.29, 147.57, 147.96, 148.20, 149.40, 150.34, 158.36 (b) ppm (alles quarternäre nicht aliphatische Kohlenstoffatome) UV/VIS (CHCl₃): schwaches Absorptionsmaximum bei 435 nm
Beispiel E: m = 20; n = 1; R¹, R³ - R⁸ = H, 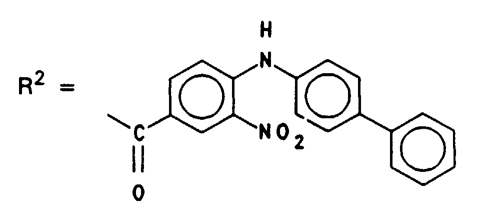 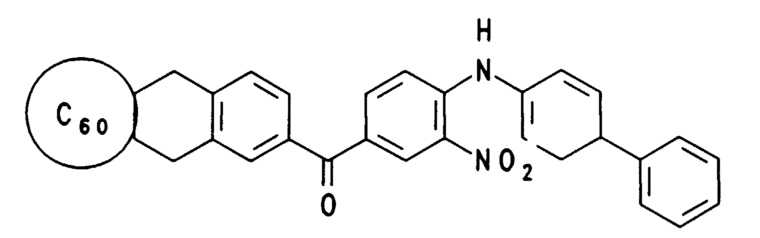 11 mg (1,1·10⁻² mmol) des Fluor-Nitro-Derivates aus Beispiel C.3 wurden in 7 ml trockenem Dimethylacetamid gelöst und mit 59.1 mg (0,35 mmol) 4-Aminobiphenyl versetzt. Die Reaktionsmischung wurde eine Woche lang auf 65°C erhitzt und anschließend mit 30 ml Waser versetzt. Hierbei fiel nach einiger Zeit ein flockiger, brauner Niederschlag aus, welcher über einen 0,25 µm Teflonmembranfilter abfiltriert wurde. Der Filterrückstand wurde mit Chloroform aus dem Filter eluiert und chromatographisch aufgearbeitet. Die Ausbeute war nahezu quantitativ.
   ¹H-NMR (500 MHz, C₂D₂Cl₄, 28°C): δ = 4.48 (b, 2H, CH₂, a), 4.82 (b, 2H, CH₂, a), 7.3-7.43 (m, 6H, CH, b, c, d, e), 7.57 (d, 2H, CH, 7.3 Hz, f), 7.65 (d, 2H, CH, 8.4 Hz, g), 7.78 Hz, h), 7.89 (dd, 1H, CH, 1.5 Hz und 8.0 Hz, i), 7.98 (dd, 1H, CH, 2 Hz und 8.0 Hz, j), 8.02 (s, 1H, CH, k), 9.85 (d, 1H, CH, 2 Hz, I)
   ¹³C-NMR (J-moduliertes Spinecho für ¹³C; 500 MHz, C₂D₂Cl₄, 28°C): δ = 48,04, 48.18 (CH₂), 68.63, 68.68 (quarternäre aliphatische C₆₀-Atome), 119.3, 128.42, 128.49, 130.11, 131.4, 131.7, 132.12, 132.23, 132.65, 133.62, 139.87 (alle CH), 129.75, 134.96, 139.61, 139.99, 141.06, 141.8, 142.77, 142.86, 143.24 (b, mehrere Signale überlagert), 144.66 (b, mehrere Signale überlagert), 145. 11, 145.21, 145.25, 145.61, 146.16 (b, mehrere Signale überlagert), 146.27, 147.72, 147.77, 148.51, 149.01, 149.29 (b, merhere Signale überlagert), 149.54 (b, mehrere Signale überlagert), 150.73, 150.76 (alles quarternäre C-Atome), 196.37 (C=O)
   UV/VIS: schwaches Absorptionsmaximum bei 430 nm
   FD-Massenspektrum: M/Z = 1139.6 (M⁺, 100 %), 720 (M⁺ von C₆₀, 5 %), 570.2 (M²⁺, 15 %)

## Patentansprüche

1. Fullerenderivat der Formel I, 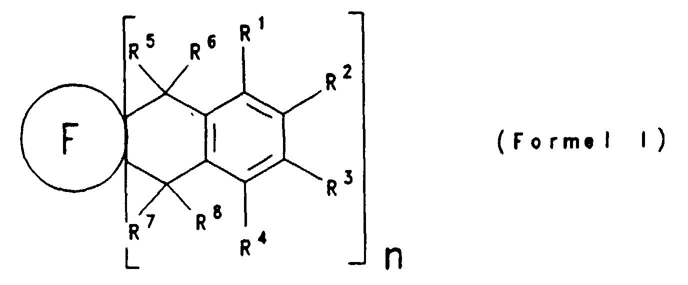 wobei die Symbole und Indices folgende Bedeutung haben:
Ⓕ ist ein Fullerenrest der Formel (C₂₀₊₂ₘ), wobei m die Zahl 1 bis 50 darstellt
R¹ bis R⁸ ist gleich oder verschieden H, NH₂, CO₂R⁹, CN, OCOR¹⁰, COR¹⁰, Cl, Br, J, F, OR¹¹, CONH₂, C₁-C₂₀ Alkyl, das mit Cl, J, Br und F substituiert sein kann, C₃-C₈ Cycloalkyl, Aryl, Heteroaryl, wobei R⁹ bis R¹¹ H, C₁-C₂₀ Alkyl, welches durch F, Cl, Br oder J substituiert sein kann, Pyridinyl oder Phenyl welches seinerseits durch F, Cl, Br, J, Nitro, Amino, C₁-C₂₀-Alkylamin, C₆-C₁₄-Arylamin, C₁-C₂₀-Alkoxy oder C₆-C₁₄-Aryloxy substituiert sein kann, oder -(CH₂)ⱼCO₂H mit j = 1 bis 10 ist, R¹-R⁴ und/oder R⁵,R⁷ können auch Teil eines cyclo-aliphatischen, aromatischen oder heteroaromatischen Systems sein, welches seinerseits mit C₁-C₂₀ Alkyl, Aryl, Carboxyl, Carbonyl, Alkoxy, Aryloxy, F, Cl, Br, J, Nitro-, Alkohol oder Amin substituiert ist, oder R¹ und R², R² und R³, R³ und R⁴ können jeweils gemeinsam 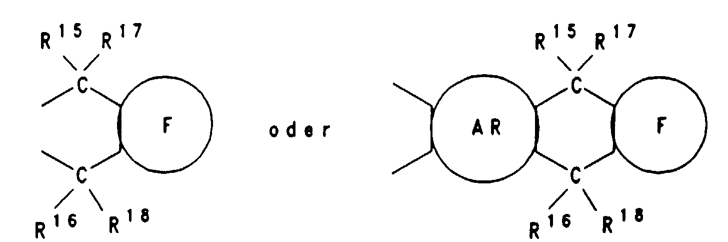 sein, wobei R¹⁵ - R¹⁸ H, C₁-C₂₀ Alkyl, F, Cl, Br, J oder Phenyl, und 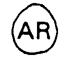 den Rest eines annelierten aromatischen Systems darstellen, R⁵ bis R⁸ kann auch das Strukturelement der Formel V 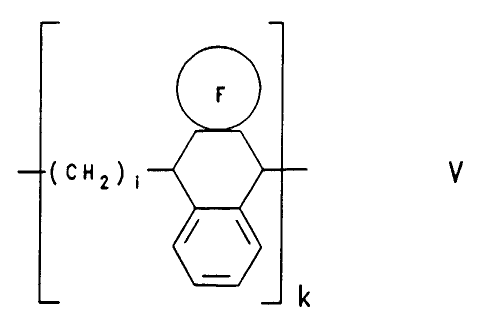 sein, in der eine Zahl von 2 bis 20 und k eine Zahl größer 1 ist, und R⁵ und R⁷ zusammen auch eine Brücke aus -O- sein können,
n ist 1 bis 20.

2. Fullerenderivat nach Anspruch 1, wobei die Symbole und Indices die folgende Bedeutung haben:
Ⓕ ist ein Fullerenderivat der Formel (C₂₀₊₂ₘ), in dem m 20, 25, 28, 29, 31 oder 32 ist,
n ist 1 oder 2.

3. Fullerenderivat nach Anspruch 1, wobei die Symbole und Indices die folgende Bedeutung haben:
Ⓕ ist C₆₀ oder C₇₀
R¹ bis R⁴ sind gleicht oder verschieden und bedeuten
H, NH₂, COR⁹, CO₂R¹⁰, -O-R¹¹, OCOR¹⁰, oder C₁-C₁₀ Alkyl, das mit F, Cl, Br oder J substituiert sein kann, wobei R⁹ bis R¹¹ H, (C₁-C₁₀)-Alkyl, das mit F, Cl oder Br substituiert sein kann, Pyridinyl, Phenyl, welches durch F, Cl, Br, Nitro, Amino, C₁-C₁₀-ASky-amin, C₆-C₁₄-Arylamin, C₁-C₁₀-Alkoxy oder C₆-C₁₄-Aryloxy substituiert sein kann,
oder -(CH₂); -CO₂H mit j = 1 bis 10 ist oder R² und R³ zusammen 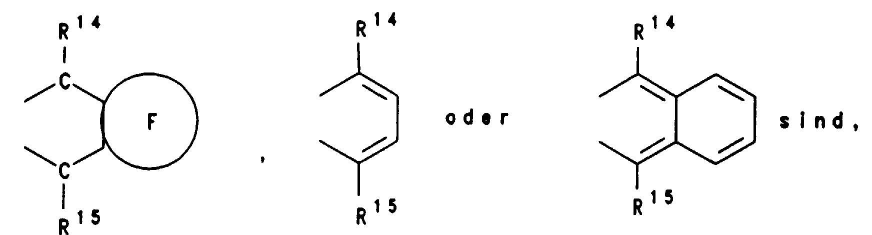 wobei R¹⁴ und/oder R¹⁵ H, Phenyl oder (C₁-C₁₀)-Alkyl ist, und
R⁵ bis R⁸ ist gleich oder verschieden H, F, Cl, Br, (C₁-C₁₂)-Alkyl oder ein Strukturelement der Formel V mit i = 4 bis 12 und k eine Zahl größer 1 und
n ist 1 oder 2.

4. Fullerenderivat nach Anspruch 3, dadurch gekennzeichnet, daß Ⓕ C₆₀ ist.

## Claims

1. A fullerene derivative of the formula I, 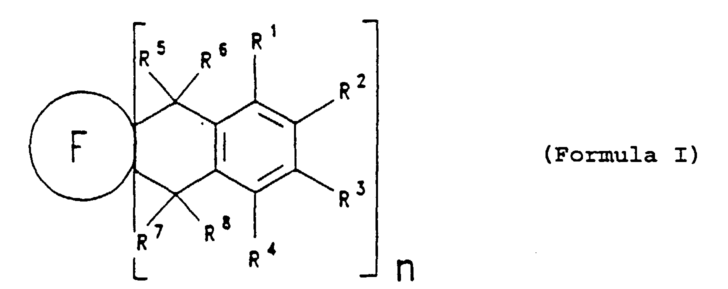 where the symbols and indices have the following meanings:
Ⓕ is a fullerene radical of the formula (C₂₀₊₂ₘ), where m is a number from 1 to 50
R¹ to R⁸ are identical or different and are each H, NH₂, CO₂R⁹, CN, OCOR¹⁰, COR¹⁰, Cl, Br, I, F, OR¹¹, CONH₂ C₁-C₂₀-alkyl which can be substituted by Cl, I, Br and F, C₃-C₈-cycloalkyl, aryl, heteroaryl, where R⁹ to R¹¹ are each H, C₁-C₂₀-alkyl which can be substituted by F, Cl, Br or I, pyridinyl or phenyl which in turn can be substituted by F, Cl, Br, I, nitro, amino, C₁-C₂₀-aCkySamine, C₆-C₁₄-arylamine, C₁-C₂₀-alkoxy or C₆-C₁₄-aryloxy, or -(CH₂)ⱼ-CO₂H having j = 1 to 10, R¹-R⁴ and/or R⁵, R⁷ can also be part of a cycloaliphatic, aromatic or heteroaromatic system which is in turn substituted by C₁-C₂₀-alkyl, aryl, carboxyl, carbonyl, alkoxy, aryloxy, F, Cl, Br, I, nitro, alcohol or amine, or R¹ and R², R² and R³, R³ and R⁴ can in each case together be 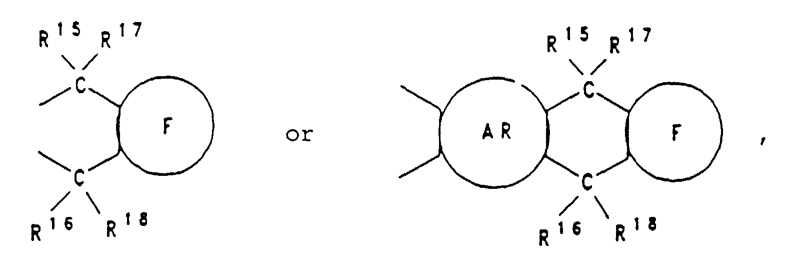 where R¹⁵-R¹⁸ are each H, C₁-C₂₀-alkyl, F, Cl, Br, I or phenyl, and 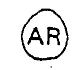 is the radical of a fused aromatic system, R⁵ to R⁸ can also be the structural element of the formula V 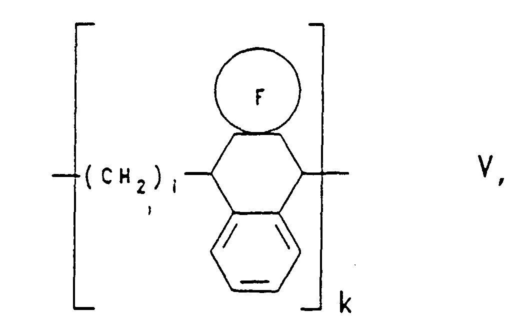
where i is a number from 2 to 20 and k is a number greater than 1, and R⁵ and R⁷ can together also be an -O- bridge,
n is from 1 to 20.

2. A fullerene derivative as claimed in claim 1, wherein the symbols and indices have the following meanings:
Ⓕ is a fullerene derivative of the formula (C₂₀₊₂ₘ) in which m is 20, 25, 28, 29, 31 or 32,
n is 1 or 2.

3. A fullerene derivative as claimed in claim 1, wherein the symbols and indices have the following meanings:
Ⓕ is C₆₀ or C₇₀
R¹ to R⁴ are identical or different and are each H, NH₂, COR⁹, CO₂R¹⁰, -O-R¹¹, OCOR¹⁰ or C₁-C₁₀-alkyl which can be substituted by F, Cl, Br or I, where R⁹ to R¹¹ are each H, (C₁-C₁₀)-alkyl which can be substituted by F, Cl or Br, pyridinyl, phenyl which can be substituted by F, Cl, Br, nitro, amino, C₁-C₁₀-alkylamine, C₆-C₁₄-arylamine, C₁-C₁₀-alkoxy or C₆-C₁₄-aryloxy,
or -(CH₂)ⱼ-CO₂H having j = 1 to 10, or R² and R³ together are 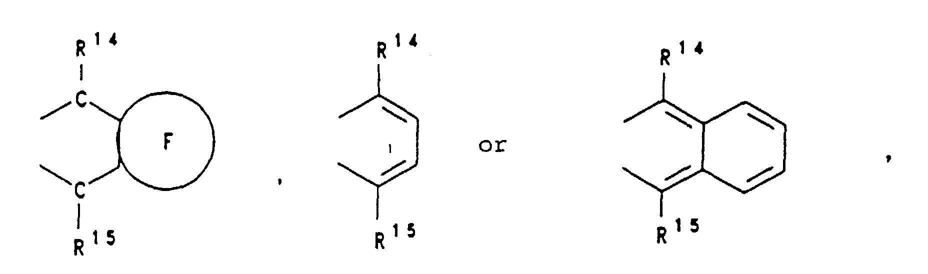 where R¹⁴ and/or R¹⁵ are each H, phenyl or (C₁-C₁₀)-alkyl, and
R⁵ to R⁸ are identical or different and are each H, F, Cl, Br, (C₁-C₁₂-alkyl or a structural element of the formula V having i = 4 to 12 and k being a number greater than 1 and
n is 1 or 2.

4. A fullerene derivative as claimed in claim 3, wherein Ⓕ is C₆₀.

## Revendications

1. Dérivé de fullerène de formule I 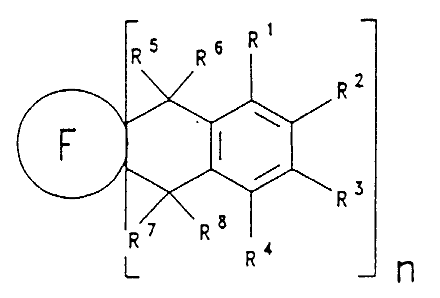 dans laquelle les symboles et indices ont les significations suivantes :
Ⓕ est un radical fullerène de formule (C₂₀₊₂ₘ), où m est un nombre de 1 à 50,
R¹ à R⁸ sont identiques ou différents et représentent chacun H, NH₂, CO₂R⁹, CN, OCOR¹⁰, COR¹⁰, Cl, Br, I, F, OR¹¹, CONH₂, un radical alkyle en C₁-C₂₀ pouvant être substitué par Cl, I, Br et F, cycloalkyle en C₃-C₈, aryle, hétéroaryle, où R⁹ à R¹¹ sont H, des radicaux alkyle en C₁-C₂₀ pouvant être substitués par F, CI, Br ou I, pyridinyle ou phényle, qui pour sa part peut être substitué par des substituants F, CI, Br, I, nitro, amino, alkylamine en C₁-C₂₀, arylamine en C₆-C₁₄, alcoxy en C₁-C₂₀ ou aryloxy en C₆-C₁₄, ou encore -(CH₂)ⱼ-CO₂H où j vaut de 1 à 10, R¹ à R⁴ et/ou R⁵, R⁷ peuvent aussi représenter une partie d'un système cycloaliphatique, aromatique ou hétéroaromatique, qui pour sa part peut être substitué par des substituants alkyle en C₁-C₂₀, aryle, carboxyle, carbonyle, alcoxy, aryloxy, F, Cl, Br, I, nitro, alcool ou amine, ou encore chacun des
R¹ et R², R² et R³, R³ et R⁴, peut former ensemble 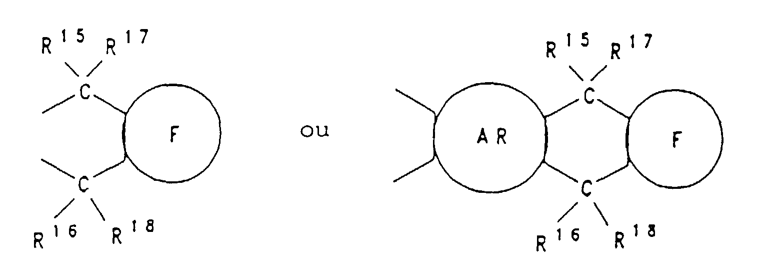
où R¹⁵-R¹⁸ sont H, des radicaux alkyle en C₁-C₂₀, F, Cl, Br, I ou phényle, et 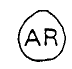 représente le résidu d'un système aromatique condensé,
R⁵ à R⁸ peuvent aussi représenter l'élément, structural de formule V 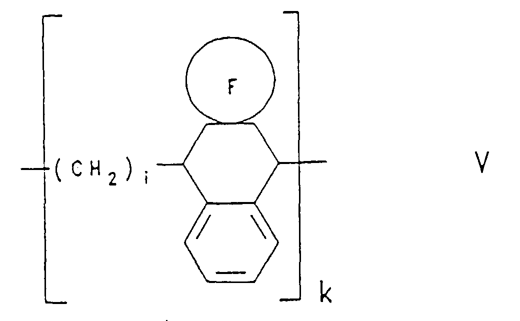 dans laquelle i est un nombre de 2 à 20 et k est un nombre supérieur à 1, et R⁵ et R⁷ peuvent aussi former ensemble un pont constitué de -O-,
n vaut 1 à 20.

2. Dérivé de fullerène selon la revendication 1, dans lequel les symboles et indices ont les significations suivantes :
Ⓕ est un dérivé de fullerène de formule (C₂₀₊₂ₘ), où m vaut 20, 25, 28, 29, 31 ou 32,
n vaut 1 ou 2.

3. Dérivé de fullerène selon la revendication 1, dans lequel les symboles et indices ont les significations suivantes :
Ⓕ est un fullerène en C₆₀ ou C₇₀,
R¹ à R⁴ sont identiques ou différents et représentent chacun H, NH2, COR⁹, CO₂R¹⁰, -O-R¹¹, OCOR¹⁰ ou un radical alkyle en C₁-C₁₀, qui peut être substitué par F, Cl, Br ou I, où R⁹ à R¹¹ représentent H, des radicaux alkyle en C₁-C₁₀ pouvant être substitués par F, CI ou Br, pyridinyle, phényle, pouvant être substitué par F, Cl, Br , nitro, amine, alkylamine en
C₁-C₁₀, arylamine en C₆-C₁₄, alcoxy en C₁-C₁₀ ou aryloxy en C₆-C₁₄,
ou encore -(CH₂)ⱼ-CO₂H avec j = 1 à 10, ou encore R² et R³ forment ensemble 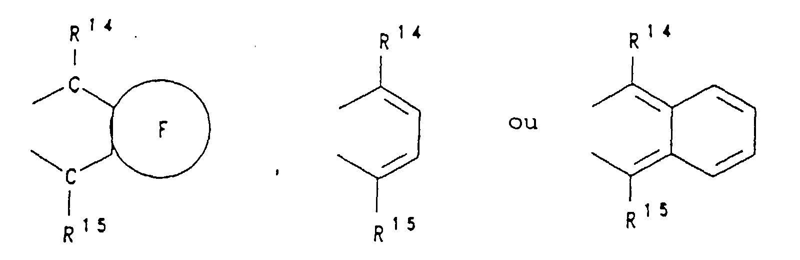
où R¹⁴ et/ou R¹⁵ sont H phényle ou alkyle en C₁-C₁₀, et
R⁵ à R⁸ sont identiques ou différents et représentent H, F, Cl, Br, des radicaux alkyle en C₁-C₁₂ ou un élément structural de formule V dans lequel i vaut de 4 à 12 et k est un nombre supérieur à 1, et
n vaut 1 ou 2.

4. Dérivé de fullerène selon la revendication 3, caractérisé en ce que Ⓕ est C₆₀.
